(19) Europäisches Patentamt
European Patent Office
Office européen des brevets

(11) Veröffentlichungsnummer: **0 184 732**
**A2**

(12) **EUROPÄISCHE PATENTANMELDUNG**

(21) Anmeldenummer: **85115157.1**

(22) Anmeldetag: **29.11.85**

(51) Int. Cl.⁴: **C 07 C 103/66**, C 07 C 102/00

(30) Priorität: **08.12.84 DE 3444915**

(43) Veröffentlichungstag der Anmeldung: **18.06.86 Patentblatt 86/25**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Keller, Reinhold, Dr., Wiesenweg 5, D-6232 Bad Soden am Taunus (DE)**

(54) **Verwendung von N-Maleyl-phenylalaninalkylester, und ein Verfahren zu seiner Herstellung.**

(57) N-Maleyl-phenylalaninalkylester wird hergestellt, indem die Ausgangsprodukte, L-Phenylalaninalkylester und Maleinsäureanhydrid, in wäßriger Lösung bei einem pH-Wert von höchstens 7,5 umsetzt. Die Reaktionsprodukte können mit dipolar aprotischen Lösemitteln in die entsprechenden Fumar-Monoamide isomerisiert werden und können somit als Zwischenprodukt bei der Herstellung von α-L-Aspartyl-L-phenylalaninalkylester dienen.

EP 0 184 732 A2

HOECHST AKTIENGESELLSCHAFT HOE 84/F 292 Dr.KH/mü

# Verwendung von N-Maleyl-phenylalaninalkylester, und ein Verfahren zu seiner Herstellung

In der veröffentlichten japanischen Patentanmeldung 49-42491 (1974) ist beschrieben, daß man α-L-Aspartyl-L-phenylalaninalkylester, die sich als Süßstoffe eignen, durch chemische bzw. biochemische Aminierung in α-Position aus den entsprechenden Fumarylmonophenylalaninalkylestern herstellen kann. Diese Fumaryl-Verbindungen werden durch Umsetzung von Fumarsäuredichlorid mit höchstens äquimolaren Mengen an Phenylalaninalkylestern in organischen Lösemitteln hergestellt und die entsprechenden Monosäurechloride anschließend hydrolysiert. Im Falle des Fumarylmono-L-phenylalaninmethylesters beträgt die Ausbeute maximal 62 %. Der Fumaryldiphenylalaninalkylester soll nur in kleinen Mengen entstehen. Wegen des Ausgangsmaterials Fumarsäuredichlorid und der relativ geringen Ausbeute ist dieses Verfahren unbefriedigend.

Überraschend wurde nun gefunden, daß N-Maleylphenylalaninalkylester in sehr hoher Ausbeute in das entsprechende Fumarsäurederivat isomerisiert werden kann. Die Maleinsäure-Monoamide eignen sich somit als Zwischenprodukte zur Herstellung der entsprechenden Fumarsäure-Monoamide. Sie können aber auch direkt durch chemische oder biochemische Aminierung nach an sich bekannten Methoden in die entsprechenden Aspartylverbindungen überführt werden.

Die Erfindung betrifft somit die Verwendung der Verbindung der allgemeinen Formel I,

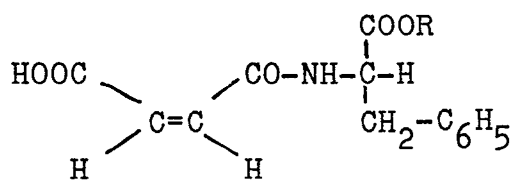

I

in der R Alkyl mit 1 bis 4 Kohlenstoffatomen bedeutet, zur Herstellung der entsprechenden Fumarsäure-Monoamide der allgemeinen Formel II,

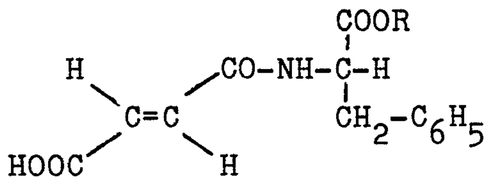 II

in der R die obengenannte Bedeutung hat, durch Isomerisierung.

In der veröffentlichten japanischen Patentanmeldung Sho 48-52 741 wird ein Herstellungsverfahren von N-Maleyl-phenylalaninalkylester beschrieben. Man erhält dieses Produkt, indem L-Phenylalaninalkylester und Maleinsäureanhydrid in einem organischen Lösemittel bei Zimmertemperatur zur Reaktion gebracht werden. Diese Reaktion verläuft jedoch nur in Gegenwart stöchiometrischer Mengen von Triethylamin und mit einer Gesamtausbeute von 85 %. Eingesetztes Triethylamin geht dabei als Ammoniumchlorid verloren und muß entsorgt werden.

Es wurde nun gefunden, daß L-Phenylalaninalkylester und Maleinsäureanhydrid auch in wäßriger Lösung zur Reaktion gebracht werden können. Dabei entsteht N-Maleyl-phenylalaninalkylester in praktisch quantitativer Ausbeute. Dies ist insbesondere insofern überraschend, da man in wäßriger Lösung, vor allem im sauren pH-Bereich, eine Hydrolyse des Esters befürchtete.

Die Erfindung betrifft somit auch ein Verfahren zur Herstellung von N-Maleyl-phenylalaninalkylester der allgemeinen Formel I, das dadurch gekennzeichnet ist, daß man L-Phenylalaninalkylester der allgemeinen Formel III

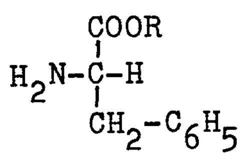 III

in der R Alkyl mit 1 bis 4 Kohlenstoffatomen bedeutet mit Maleinsäureanhydrid in wäßriger Lösung bei einem pH-Wert von höchstens 7,5 umsetzt.

Als L-Phenylalanin-alkylester der Formel III wird bevorzugt der Methylester bzw. der Ethylester verwendet. Die Ester können in freier Form eingesetzt und dann unter guter Durchmischung in Wasser fein verteilt werden. Durch Einstellen eines pH-Wertes im Bereich von etwa 3 bis 7,5 werden sie in Lösung gebracht. Vorteilhafter ist es, den Ester der Formel III in Form seines Salzes, beispielsweise des Hydrochlorids, einzusetzen, da die Salze leichter löslich sind. Zu dieser Lösung wird nun nach und nach das Maleinsäureanhydrid zugegeben, wobei diese Zugabe kontinuierlich oder in Intervallen erfolgen kann. Durch Zugabe einer geeigneten Base, zweckmäßig einer Alkalilauge wie Natronlauge, wird der pH-Wert in dem genannten Bereich gehalten, vorteilhaft bei pH 6,0, da hier die Umsetzung besonders rasch und vollständig verläuft.

Es ist möglich, einen der beiden Reaktionsteilnehmer im Überschuß einzusetzen, beispielsweise das Maleinanhydrid. Da aber auch beim äquimolaren Einsatz die Umsetzung rasch und vollständig verläuft, werden hierdurch im allgemeinen keine Vorteile erlangt.

Das Maleinsäureanhydrid kann in fester Form oder in Lösung zudosiert werden. Als Lösemittel ist Wasser möglich, bevorzugt sind inerte polare organische Lösemittel, und zwar einerseits wassermischbare wie Aceton, andererseits wenig wasserlösliche wie Methyl-isobutyl-keton, das auch zur Extraktion des Produktes dienen kann.

Die Umsetzung kann in einem Bereich von etwa 0 bis 50°C erfolgen, vorteilhaft bei 10 bis 40°C, insbesondere bei Raumtemperatur.

Nach beendeter Umsetzung kann das Produkt durch Ansäuern auf einen pH-Wert von 3 oder darunter ausgefällt und/oder mit einem organischen Lösemittel extrahiert werden.

Die Verfahrensprodukte werden in nahezu quantitativer Ausbeute und hoher chemischer und optischer Reinheit erhalten.

Die Isomerisierung zu den entsprechenden Fumarsäure-Halbamiden kann nach üblichen Verfahren, beispielsweise durch Erhitzen in Gegenwart eines sauren Katalysators, etwa einer Mineralsäure, erfolgen, wobei die entsprechenden Vorkehrungen getroffen werden müssen, damit beispielsweise eine Verseifung des Esters oder andere Nebenreaktionen unterbleiben. Besonders vorteilhaft ist die Isomerisierung in inerten dipolaren aprotischen Flüssigkeiten, wie sie in der britischen Patentschrift 1 246 349 beschrieben ist. Bei diesem Isomerisierungsverfahren kann die Verbindung der Formel I in dem inerten dipolaren aprotischen Lösemittel gelöst sein, oder aber als Lösung in einem anderen Lösemittel, beispielsweise einem unpolaren organischen Lösemittel, eingesetzt werden. Eine solche Lösung in einem unpolaren organischen Lösemittel kann beispielsweise der Extrakt der Verbindung der Formel I aus dem Reaktionsmedium sein, der nötigenfalls getrocknet wurde. Bezüglich der Einzelheiten wird auf die britische Patentschrift 1 246 349 verwiesen.

Der so hergestellte Fumarylmono-L-phenylalaninalkylester kann dann zum α-L-Aspartyl-L-phenylalaninalkylester umgesetzt werden.

In den folgenden Beispielen wird die Erfindung näher erläutert.

Beispiel 1

Herstellung von N-Maleyl-L-phenylalaninmethylester

215 g (1 Mol) L-Phenyalaninmethylester-Hydrochlorid werden in 1 l Wasser gelöst und der pH-Wert der Lösung durch Zugabe von 5 N-Natronlauge auf 6,0 eingestellt. Unter Rühren gibt man portionsweise 98 g (1 Mol) pulverförmiges Maleinsäureanhydrid zu und hält dabei den pH-Wert der Reaktionslösung durch Zugabe von 5 N-Natronlauge auf 6,0. Nach beendeter Zugabe rührt man noch weitere 10 Minuten nach und stellt dann den pH-Wert der Lösung mit konzentrierter Salzsäure auf 3,0 oder darunter. Das in Form eines Öls ausgefallene Produkt wird in Diethylether aufgenommen, die Lösung mit Magnesiumsulfat getrocknet und eingeengt. Man erhält 271 g (98 % d. Th.) farblose Kristalle vom Schmelzpunkt 90°C.

Spezifischer Drehwert: $[\alpha]_D^{25}$ + 148,9 (c= 2, $CHCl_3$)

Elementaranalyse: $C_{14}H_{15}O_5N$ (277)

| | C | H | N | % |
|---|---|---|---|---|
| ber.: | 60,6 | 5,5 | 5,1 | |
| gef.: | 60,3 | 5,3 | 5,1 | |

In analoger Weise können auch die anderen niederen Alkylester, beispielsweise der Ethylester, hergestellt werden.

Beispiel 2

Herstellung von N-Fumaryl-L-phenylalaninmethylester

50 g des nach Beispiel 1 erhaltenen N-Maleylhalbamids werden in 200 ml Xylol und 10 ml Dimethylformamid aufgenommen und 8 Stunden unter Rückfluß gekocht. Die Lösung wird auf Raumtemperatur abgekühlt, die dabei ausgefallenen Kristalle abfiltriert und mit Diethylether nachgewaschen.

Man erhält 48 g (96 % d. Th.) farblose Kristalle vom Schmelzpunkt 153°C.

Spezifischer Drehwert: $[\alpha]_D^{25}$ + 56,1 (c= 2, $CHCl_3$)

In analoger Weise können auch die anderen niederen Alkylester isomerisiert werden.

<u>Patentansprüche:</u>

1. Verwendung der Verbindung der allgemeinen Formel I,

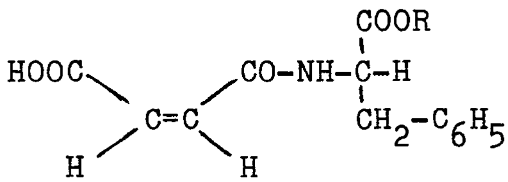

I

in der R Alkyl mit 1 bis 4 Kohlenstoffatomen bedeutet, zur Herstellung der Verbindung der allgemeinen Formel II,

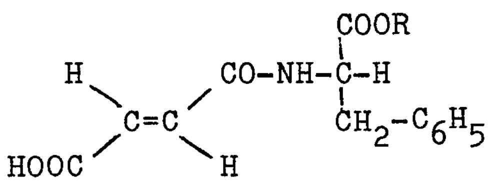

II

in der R die obengenannte Bedeutung hat, durch Isomerisierung.

2. Verwendung der Verbindung der allgemeinen Formel I gemäß Anspruch 1 zur Herstellung der entsprechenden α-L-Aspartyl-L-phenylalaninalkylester.

3. Verwendung der Verbindung der allgemeinen Formel I gemäß Anspruch 1, dadurch gekennzeichnet, daß R Methyl oder Ethyl bedeutet.

4. Verfahren zur Herstellung der Verbindungen der allgemeinen Formel I gemäß Anspruch 1, dadurch gekennzeichnet, daß man L-Phenylalaninalkylester der allgemeinen Formel III,

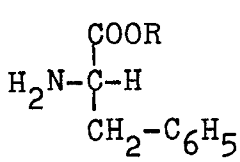

III

in der R Alkyl mit 1 bis 4 Kohlenstoffatomen bedeutet, in wäßriger Lösung in einem pH-Bereich von 3 bis 7,5 mit Maleinsäureanhydrid umsetzt.

5. Verfahren nach Anspruch 4, dadurch gekennzeichnet, daß die Umsetzung bei 0 bis 50°C erfolgt.

6. Verfahren nach Anspruch 5, dadurch gekennzeichnet, daß die Umsetzung bei 10 bis 40°C erfolgt.

7. Verfahren nach einem oder mehreren der Ansprüche 4 bis 6, dadurch gekennzeichnet, daß der L-Phenylalaninalkylester in Form seiner Salze eingesetzt wird.

8. Verfahren nach Anspruch 7, dadurch gekennzeichnet, daß der L-Phenylalaninalkylester in Form seiner Hydrochloride eingesetzt wird.